# EUROPÄISCHE PATENTANMELDUNG

(11) **EP 4 049 608 A1**
(43) Veröffentlichungstag der Anmeldung: **31.08.2022**
(21) Anmeldenummer: 22153710.3
(22) Anmeldetag: 27.01.2022
(51) Int. Cl.: A61B 34/00, A61B 34/30, A61B 17/29

(54) **INSTRUMENT FÜR EIN ROBOTISCHES OPERATIONSSYSTEM**

(30) Priorität: 25.02.2021 DE 102021104516
(71) Anmelder: avateramedical GmbH, 07745 Jena (DE)
(72) Erfinder: MARKWEG, Eric, 98693 Ilmenau (DE); BAUR, Thomas, 72108 Rottenburg (DE); BURKHARD, Manfred, 86911 Dießen (DE)
(74) Vertreter: Gleim Petri Oehmke Patent- und Rechtsanwaltspartnerschaft mbB

(57) **Zusammenfassung**

Die Erfindung betrifft ein Instrument für ein robotisches Operationssystem. Dieses Instrument umfasst ein Gehäuse (1) und einen äußeren Schaft (2), welcher an einem distalen Ende mit einem Effektor (3) und an einem proximalen Ende mit dem Gehäuse (1) über ein fest mit dem äußeren Schaft (2) verbundenes erstes Koppelelement (4) rotatorisch zur Rotation des äußeren Schaftes (2) um eine Achse verbunden ist. Das Instrument umfasst außerdem einen ersten inneren Schaft (7), welcher teilweise innerhalb des äußeren Schaftes (2) angeordnet und relativ zum äußeren Schaft (2) beweglich gelagert ist. Der erste innere Schaft (7) ist an einem proximalen Ende fest mit einem zweiten Koppelelement (8) verbunden.

Bei einem solchen Instrument umschließt das erste Koppelelement (4) den ersten inneren Schaft (7) in einem ersten Übergangsbereich (26) am proximalen Ende des äußeren Schaftes (2), und ein erstes Dichtungselement (23) umschließt den ersten inneren Schaft (7). Dabei ist es an einem proximalen Ende des ersten Koppelelements (4) fixiert oder innerhalb des ersten Koppelelements (4) im ersten Übergangsbereich (26) angeordnet und axial von dem proximalen Ende des äußeren Schaftes (2) und dem ersten Koppelelement (4) fixiert und dichtet den ersten inneren Schaft gegen den äußeren Schaft (2) ab.

## Beschreibung

### Gebiet der Erfindung

Die Erfindung betrifft ein Instrument für ein robotisches Operationssystem, welches ein Gehäuse umfasst sowie einen äußeren Schaft, welcher an einem distalen Ende mit einem Effektor und an einem proximalen Ende mit dem Gehäuse verbunden ist. Die Verbindung ist dabei rotatorisch, so dass der äußere Schaft um eine Achse - seine Längsachse - relativ gegenüber dem Gehäuse rotieren kann. Die rotatorische Verbindung erfolgt dabei über ein fest mit dem äußeren Schaft verbundenes erstes Koppelelement. Das Instrument umfasst außerdem einen ersten inneren Schaft, welcher teilweise innerhalb des äußeren Schaftes angeordnet und relativ zum äußeren Schaft beweglich gelagert ist. Bei der Relativbewegung kann es sich um eine Rotation oder eine Translation handeln. Der erste innere Schaft ist an einem distalen Ende mit dem Effektor und an einem proximalen Ende fest mit einem zweiten Koppelelement verbunden.

### Stand der Technik

Ein solches Instrument für die minimal-invasive Chirurgie wird beispielsweise in der DE 10 2014 117 408 A1 der Anmelderin beschrieben. Bei Instrumenten mit mehreren gegeneinander translatorisch oder rotatorisch beweglichen Schäften besteht immer die Gefahr, dass Körperflüssigkeiten und/oder Gewebe des Patienten in den Bereich der Antriebseinheit des Instruments eindringen und somit eine Kontamination des robotischen Operationssystems verursachen.

Im Stand der Technik sind bereits verschiedene Instrumente bekannt, welche zu diesem Zweck Dichtungen aufweisen. In der US 2012/0209289 A1 wird ein Instrument beschrieben, bei dem die Antriebswelle von einem O-Ring umschlossen wird, wobei der O-Ring formschlüssig in einer Aussparung eines Gehäuseteils gehalten wird und so gegen axiales Verschieben gesichert ist. In der US 7,367,973 B2 wird ein Instrument beschrieben, bei dem im Bereich des Effektors, also am distalen Ende des Schaftes, zwei O-Ringe angeordnet sind, einer der O-Ringe dabei in einer speziellen Aufnahme, die ihn gegen axiales Verschieben sichert. Auch die US 2017/0290680 A1 beschreibt die Verwendung eines O-Rings zur Abdichtung, welcher im Schaft des Instruments formschlüssig gehalten wird.

In der US 2016/0193001 A1 wird die Verwendung eines Silikonrings im Bereich des Effektors zwischen der Abschlusskappe und dem Gabelkopf bzw. zwischen dem Gabelkopf und dem Ende des Effektors beschrieben. Allerdings wird die Verwendung eines solchen Silikonrings als nachteilig beschrieben. Besser sei es, entsprechende Abstände zu minimieren. Dies würde die Herstellung vereinfachen und den Aufwand für die Wartung verringern.

In der DE 10 2007 030 856 B3 wird ein chirurgisches Instrument mit zwei gegeneinander beweglichen Schäften - hier als Kolben bezeichnet - beschrieben, zwischen denen sich ein Fluidabschnitt befindet. Zur Abdichtung wird ein Dichtungselement verwendet, welches als flexible, inelastische, fluiddichte Membran ausgebildet ist und einen Rollbalg bildet. Ein Abschnitt ist dabei hülsenförmig ausgebildet, ein zweiter Abschnitt füllt die Querschnittsfläche des Schaftes.

In der US 2008/0065021 A1 wird ein Instrument beschrieben, welches am proximalen Ende eine Dichtung, welche als Membran mit einem Schlitz ausgebildet ist, aufweist, die verhindern soll, dass Gas aus dem Operationsraum entweicht. Die WO 2019/072988 A1 beschreibt ein Endoskop mit einer doppelten Dichtung in axialer Richtung zum fluiddichten Abdichten des Arbeitskanals. Die Dichtungen sind miteinander verbunden und können zum Reinigen oder Auswechseln per Hand ohne Hilfsmittel entfernt werden.

Die US 2016/0175060 A1 beschreibt ein Instrument mit einem wiederverwendbaren Schaft und austauschbarer Spitze. Zur Abdichtung zwischen der Innenwand der Instrumentenspitze und dem Schaft wird ein hülsenförmiges Dichtelement aus Silikon verwendet, welches entlang einer Längsachse der Instrumentenspitze beweglich ist.

In der US 2010/0168510 A1 werden am distalen Ende eines Instruments Dichtungen, insbesondere in Form von O-Ringen, die in Nuten formschlüssig gehalten werden, verwendet, um den Austritt von Schmiermittel aus dem Instrument in den Behandlungsraum zu verhindern.

### Beschreibung der Erfindung

Aufgabe der Erfindung ist es, ein Instrument, wie es eingangs beschrieben wurde, dahingehend weiterzuentwickeln, dass ein Eindringen von Körperflüssigkeiten und/oder Gewebe des Patienten in den Bereich der Antriebseinheit möglichst effizient verhindert wird, wobei an einem bestehenden Instrument möglichst wenig Änderungen vorgenommen werden sollen, um den Herstellungsprozess nicht modifizieren zu müssen; so soll z.B. auf das Anbringen zusätzlicher Nuten oder Aufnahmen für Dichtringe verzichtet werden können, um insbesondere bereits vorhandene Instrumenten-Antriebseinheiten, an welche die Instrumente angekoppelt werden, weiter verwenden zu können.

Diese Aufgabe wird bei einem Instrument der eingangs beschriebenen Art dadurch gelöst, dass das erste Koppelelement den ersten inneren Schaft in einem ersten Übergangsbereich am proximalen Ende des äußeren Schafts umschließt, und ein erstes Dichtungselement den ersten inneren Schaft umschließt und gegen den äußeren Schaft abdichtet. Das Dichtungselement - beispielsweise eine Hülse oder ein Ring - ist dabei im Bereich des ersten Koppelelements angeordnet. Als flexible, dehnbare Hülse von ausreichender Länge kann das erste Dichtungselement beispielsweise teilweise das erste Koppelelement und teilweise den ersten inneren Schaft umschließen. Die Hülse kann auf das erste Koppelelement und den ersten inneren Schaft bei der Montage aufgezogen werden, ohne dass weitere Mittel zur Befestigung der Hülse nötig wären. Die Abdichtung erfolgt fluiddicht, d.h. gegen den Durchtritt von Flüssigkeiten.

Je länger die Hülse entlang der Längsachse des ersten inneren Schaftes ist, desto besser ist der Dichtungseffekt; andererseits kann dies die Relativbewegung des ersten inneren Schaftes zum äußeren Schaft - bei der es sich um eine rotatorische Bewegung handeln kann, bevorzugt aber um eine translatorische Bewegung handelt - erschweren: Auch wenn diese Bewegung motorisch angetrieben erfolgt, so kann dies zu Reibungsverlusten zwischen dem erste inneren Schaft und dem ersten Dichtungselement führen, die unter Umständen eine genaue Positionierung behindern und im schlimmsten Fall auch zu einem Reißen der Hülse führen können. Um dies zu vermeiden, ist es zweckmäßig, das erste Dichtungselement mit einer geringstmöglichen axialen Ausdehnung, beispielsweise als Dichtungsring auszugestalten, wobei dann aber auf andere Weise dafür gesorgt werden muss, dass das erste Dichtungselement den ersten inneren Schaft gegen den äußeren Schaft abdichtet.

Dabei macht man sich zunutze, dass das erste Koppelelement fest mit dem äußeren Schaft verbunden ist, gleichzeitig aber den ersten inneren Schaft im ersten Übergangsbereich so umschließt, dass der erste innere Schaft ungehindert die translatorische oder rotatorische Relativbewegung gegenüber dem äußeren Schaft durchführen kann. Dies eröffnet im Wesentlichen zwei Möglichkeiten, die sich grundsätzlich auch kombinieren lassen, um den Abdichtungseffekt zu erhöhen: In einer ersten Alternative ist das erste Dichtungselement an einem proximalen Ende des ersten Koppelelements fixiert. Diese Fixierung kann stoffschlüssig erfolgen, so dass keine gesonderten Halterungen für das erste Dichtungselement, was vorzugsweise als Dichtungsring ausgestaltet ist, erforderlich sind. Das erste Dichtungselement umschließt dann also den ersten inneren Schaft am proximalen Ende des ersten Koppelelements und verhindert so, dass Flüssigkeit oder Gewebe in das Innere des Instruments tritt und die Antriebseinheit kontaminiert. In einer zweiten Alternative ist das erste Dichtungselement innerhalb des ersten Koppelelements im ersten Übergangsbereich angeordnet. Dabei wird es axial von dem proximalen Ende des äußeren Schaftes und dem ersten Koppelelement fixiert. In diesem Fall macht man sich zu Nutze, dass der Radius des äußeren Schaftes größer als der des ersten inneren Schaftes ist und somit das erste Koppelelement im Bereich des äußeren Schaftes einen größeren Innendurchmesser aufweisen muss, als es im Bereich des - vom ersten Koppelelement ebenfalls teilweise umschlossenen - ersten inneren Schaftes nötig ist. Idealerweise bildet der Bereich, in dem sich der Innendurchmesser des ersten Koppelelements verjüngt, eine Kante und damit eine - sich vorzugsweise in radialer Richtung mit einer Flächennormalen in axialer Richtung erstreckende - Anschlagfläche für das erste Dichtungselement. Dort kann das erste Dichtungselement ebenfalls stoffschlüssig fixiert werden. Vorzugsweise wird es aber auch vom proximalen Ende des äußeren Schafts, der mit dem ersten Koppelelement fest verbunden ist, fixiert, so dass es zwischen dem proximalen Ende des äußeren Schafts und der Anschlagfläche formschlüssig fixiert, eingeklemmt ist und auf eine stoffschlüssige Verbindung verzichtet werden kann. Dies erleichtert die Montage

In einer besonders bevorzugten Ausgestaltung der ersten Alternative ist zwischen dem ersten Koppelelement und dem zweiten Koppelelement ein in der Regel mit dem Gehäuse verbundenes erstes Führungselement angeordnet. In diesem wird der erste innere Schaft geführt, was dessen Lagerung zusätzlicher Stabilität verleiht. Dabei ist das erste Dichtungselement zwischen dem ersten Koppelelement und dem ersten Führungselement angeordnet und wird von diesen axial fixiert. Eine Relativbewegung zwischen dem ersten Koppelelement und dem ersten Führungselement ist nicht möglich und der Abstand zwischen beiden entlang der Längsachse des Instruments bzw. der Schäfte ist so bemessen, dass das erste Dichtungselement genau zwischen das erste Koppelelement und das erste Führungselement passt; die axiale Fixierung ist also auch in diesem Fall formschlüssig, so dass auf eine stoffschlüssige Verbindung verzichtet werden kann. Das erste Dichtungselement kann als Dichtungsring ausgebildet sein, beispielsweise als O-Ring oder als X-Ring. Das erste Dichtungselement kann Silikon enthalten oder vollständig aus Silikon gefertigt sein, wobei das Silikon bevorzugt biokompatibel ist. Das erste Dichtungselement ist bevorzugt ringförmig mit rechteckigem Querschnitt und weist an seinem inneren Umfang - der Bohrung - und/oder an einer Stirnfläche, die in Richtung des abzudichtenden Abschnitts des Instruments, hier also an der zum ersten Koppelelement weisenden Stirnfläche, umlaufend geschlossene, im Querschnitt zahnförmige Dichtlamellen auf, welche flexibel und elastisch deformierbar sind, wodurch der Dichtungseffekt verstärkt und eine bessere Anpassung an Oberflächen mit einer höheren Rauigkeit erreicht wird.

In einer weiteren Ausgestaltung umfasst das Instrument außerdem einen zweiten inneren Schaft, welcher teilweise innerhalb des ersten inneren Schafts angeordnet ist, wobei der zweite innere Schaft sowohl relativ zum äußeren Schaft als auch relativ zum ersten inneren Schaft beweglich gelagert ist - vorzugsweise rotierbar, aber auch eine translatorisch bewegliche Lagerung ist denkbar. An einem distalen Ende ist der zweite innere Schaft ebenfalls mit dem Effektor verbunden und an einem proximalen Ende fest mit einem dritten Koppelelement. Dabei umschließt das zweite Koppelelement den zweiten inneren Schaft in einem zweiten Übergangsbereich am proximalen Ende des ersten inneren Schafts, und ein zweites Dichtungselement umschließt den zweiten inneren Schaft und dichtet ihn gegen den ersten inneren Schaft fluiddicht ab. Das zweite Dichtungselement ist dabei bevorzugt analog wie das erste Dichtungselement ausgestaltet, jedoch in der Regel mit geringfügig kleinerem Durchmesser.

In einer ersten Alternative ist das zweite Dichtungselement dabei an einem proximalen Ende des zweiten Koppelelements fixiert, beispielsweise stoffschlüssig. Eine andere Möglichkeit besteht darin, das zweite Dichtungselement innerhalb des zweiten Koppelelements im zweiten Übergangsbereich anzuordnen, wo es dann axial von dem proximalen Ende des ersten inneren Schaftes und dem zweiten Koppelelement fixiert ist, so wie es vorangehend bereits für das erste Dichtungselement beschrieben wurde; diese Ausführungen lassen sich analog auf das zweite Dichtungselement übertragen. Beide Ausgestaltungen für die Anordnung des zweiten Dichtungselements können grundsätzlich auch kombiniert werden, so dass zwei Dichtungselemente verwendet werden, was die Abdichtung gegen den Durchtritt von Fluiden weiter verbessert.

In einer bevorzugten Ausgestaltung des Instruments in der ersten Alternative für das zweite Dichtungselement, bei der der erste innere Schaft relativ zum äußeren Schaft translatorisch entlang der Achse verschiebbar gelagert ist, und bei der vorteilhaft auf eine stoffschlüssige Fixierung des zweiten Dichtungselements verzichtet werden kann, umfasst das Instrument ein erstes axiales Rückstellfederelement mit einem proximalen und einem distalen Ende, welches auf das zweite Koppelelement bei dessen translatorischer Verschiebung aus einer Grundstellung heraus eine dieser Verschiebung entgegengesetzte Federkraft ausübt. Das zweite Dichtungselement ist dabei zwischen dem zweiten Koppelelement und dem ersten axialen Rückstellfederelement angeordnet und von diesen eingespannt. Diese Einspannung erfolgt bevorzugt kraft- und gleichzeitig formschlüssig; auf eine zusätzliche Fixierung kann verzichtet werden. Auf diese Weise wird außerdem ein Anpressdruck auf das zweite Dichtungselement gegen das zweite Koppelelement ausgeübt, wodurch die Abdichtung gegen den Durchtritt von Flüssigkeiten weiter verbessert wird. Eine besonders bevorzugte Ausgestaltung dieser Variante sieht ein zweites Führungselement vor, welches zwischen dem zweiten Koppelelement und dem dritten Koppelelement angeordnet ist und in welchem der zweite innere Schaft geführt wird. Das proximale Ende des ersten axialen Rückstellfederelements liegt dabei am zweiten Führungselement oder ist optional daran - beispielsweise stoffschlüssig - befestigt. Durch das zweite Führungselement wird somit die Stabilität und Sicherheit des Instruments bei der Verwendung erhöht.

In einer weiteren Ausgestaltung des Instruments für Effektoren mit mehr Freiheitsgraden umfasst das Instrument einen dritten inneren Schaft, welcher teilweise innerhalb des zweiten inneren Schafts angeordnet und relativ zum äußeren Schaft, zum ersten inneren Schaft und zum zweiten inneren Schaft beweglich gelagert ist. An einem distalen Ende ist der dritte innere Schaft mit dem Effektor und an einem proximalen Ende fest mit einem vierten Koppelelement verbunden. Dabei umschließt das vierte Koppelelement den zweiten inneren Schaft in einem dritten Übergangsbereich am proximalen Ende des zweiten inneren Schafts. Das vierte Koppelelement selbst ist am proximalen Ende durch einteilige Fertigung dicht bzw. durch ein stoffschlüssig fixiertes Abschlusselement abgedichtet. Ein drittes Dichtungselement ist außerdem an einem distalen Ende des vierten Koppelelements fixiert, umschließt den zweiten inneren Schaft und dichtet diesen gegen den dritten inneren Schaft fluiddicht ab. Auch hier kann das dritte Dichtungselement stoffschlüssig mit dem distalen Ende des vierten Koppelelements verbunden sein. Die bereits oben für das erste Dichtungselement gemachten Ausführungen, was dessen spezielle Ausgestaltung betrifft, lassen sich analog auch auf das dritte Dichtungselement übertragen und sollen hier nicht wiederholt werden. Durch die stoffschlüssige Verbindung des dritten Dichtungselements mit dem vierten Koppelelement lässt sich ebenfalls auf zusätzliche Fixierungsmittel verzichten. Die freiliegende Anordnung behindert die Herstellung einer reversiblen Verbindung mit einer Instrumenten-Antriebseinheit durch einfaches Einklicken nicht.

In einer zweckmäßigen Ausgestaltung ist der dritte innere Schaft relativ zum äußeren Schaft, relativ zum ersten inneren Schaft und relativ zum zweiten inneren Schaft translatorisch entlang der Achse verschiebbar gelagert. In diesem Fall umfasst das Instrument besonders bevorzugt ein zweites axiales Rückstellfederelement, welches mit einem distalen Ende am dritten Koppelelement anliegt oder an diesem - beispielsweise stoffschlüssig oder formschlüssig - befestigt ist und auf das vierte Koppelelement bei dessen translatorischer Verschiebung aus einer Grundstellung heraus eine dieser Verschiebung entgegengesetzte Federkraft ausübt. Das dritte Dichtungselement ist dann zwischen dem distalen Ende des vierten Koppelelements und dem proximalen Ende des zweiten axialen Rückstellfederelements angeordnet und von diesen analog zum zweiten Dichtungselement eingespannt. Die Einspannung erfolgt aufgrund der Federkraft in der Regel zumindest kraftschlüssig und formschlüssig; das zweite - und analog das erste - axiale Rückstellfederelement wird in der Regel unter Vorspannung montiert. Eine zusätzliche Fixierung, beispielsweise mittels Stoffschluss, ist daher nicht notwendig, was die Herstellung erleichtert.

In dem dritten inneren Schaft ist zweckmäßig mindestens ein Betätigungselement für den Effektor angeordnet, welches beispielsweise als Zugseil oder Zugdraht ausgebildet ist.

Es versteht sich, dass die vorstehend genannten und die nachstehend noch zu erläuternden Merkmale nicht nur in den angegebenen Kombinationen, sondern auch in anderen Kombinationen oder in Alleinstellung einsetzbar sind, ohne den Rahmen der vorliegenden Erfindung zu verlassen.

### Kurze Beschreibung der Zeichnungen

Nachfolgend wird die Erfindung anhand von Ausführungsbeispielen unter Bezugnahme auf die beigefügten Zeichnungen, die ebenfalls erfindungswesentliche Merkmale offenbaren, noch näher erläutert. Diese Ausführungsbeispiele dienen lediglich der Veranschaulichung und sind nicht als einschränkend auszulegen. Beispielsweise ist eine Beschreibung eines Ausführungsbeispiels mit einer Vielzahl von Elementen oder Komponenten nicht dahingehend auszulegen, dass alle diese Elemente oder Komponenten zur Implementierung notwendig sind. Vielmehr können andere Ausführungsbeispiele auch alternative Elemente und Komponenten, weniger Elemente oder Komponenten oder zusätzliche Elemente oder Komponenten enthalten. Elemente oder Komponenten verschiedener Ausführungsbespiele können miteinander kombiniert werden, sofern nichts anderes angegeben ist. Modifikationen und Abwandlungen, welche für eines der Ausführungsbeispiele beschrieben werden, können auch auf andere Ausführungsbeispiele anwendbar sein. Zur Vermeidung von Wiederholungen werden gleiche oder einander entsprechende Elemente in verschiedenen Figuren mit gleichen Bezugszeichen bezeichnet und nicht mehrmals erläutert. Es zeigen:
- **Fig. 1**: eine Perspektivansicht eines Instruments für eine robotisches Operationssystem,
- **Fig. 2**: das Instrument aus **Fig. 1** in einer verkürzten Darstellung von oben,
- **Fig. 3A,**: einen Schnitt durch die Instrumentenspitze,
- **Fig. 3B**: einen Schnitt durch den Korpus des Instruments,
- **Fig. 4**: einen Ausschnitt des Korpus aus **Fig. 3B****,** und
- **Fig. 5**: eine Perspektivansicht eines Dichtelements.

### Ausführliche Beschreibung der Zeichnungen

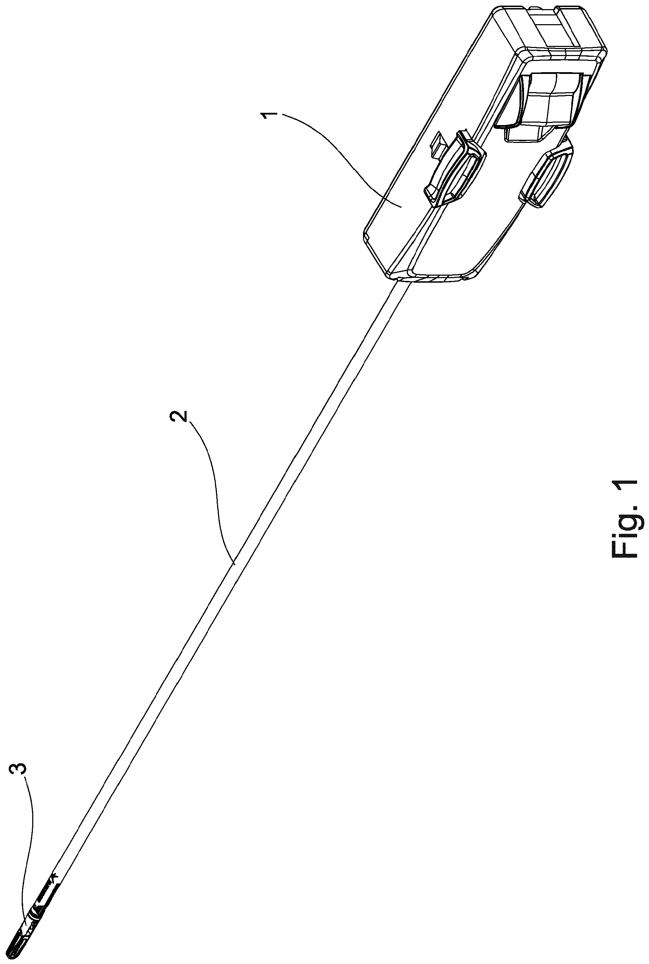

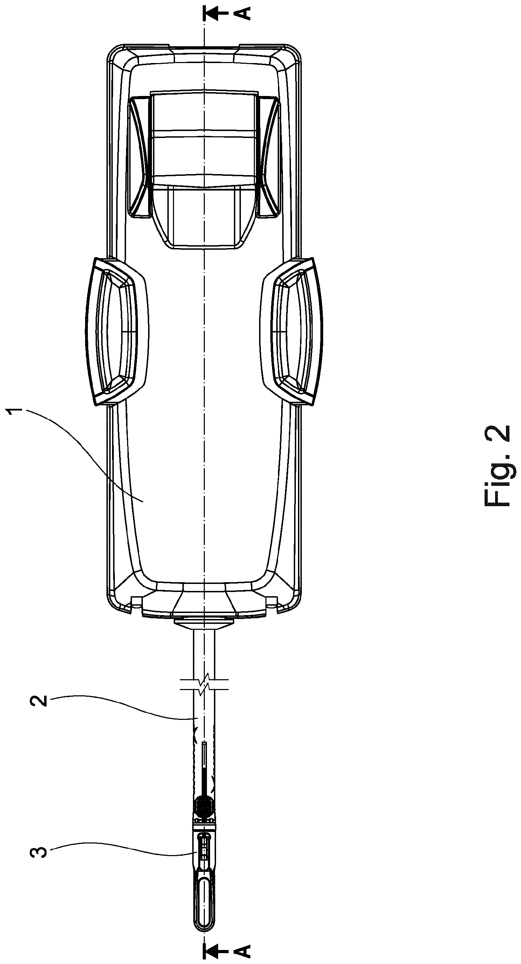

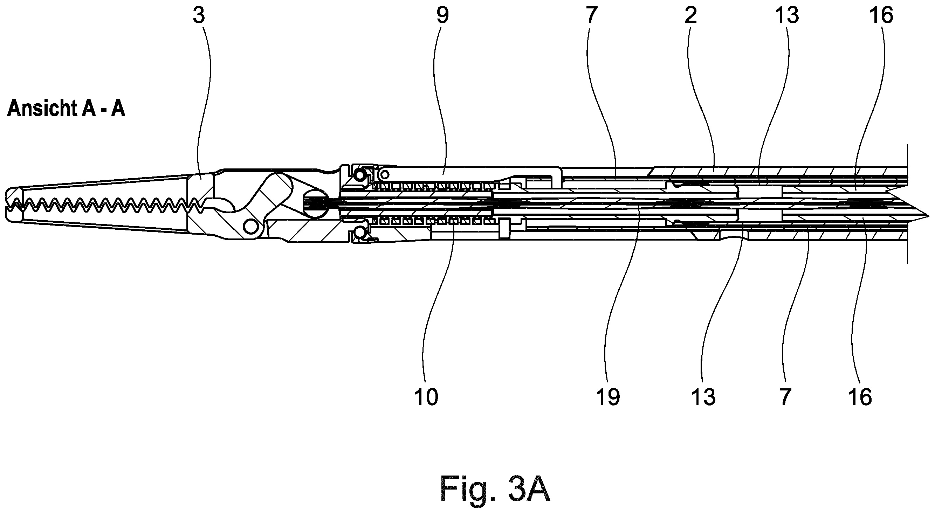

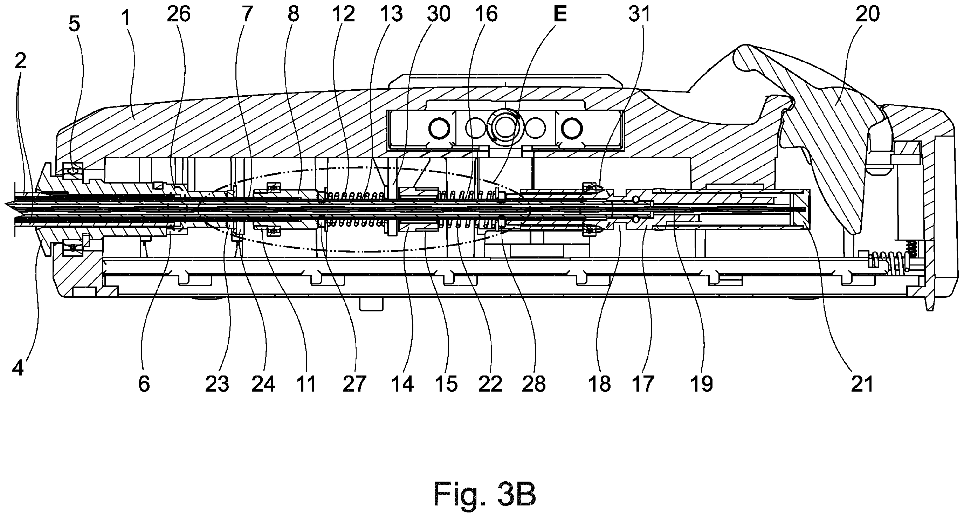

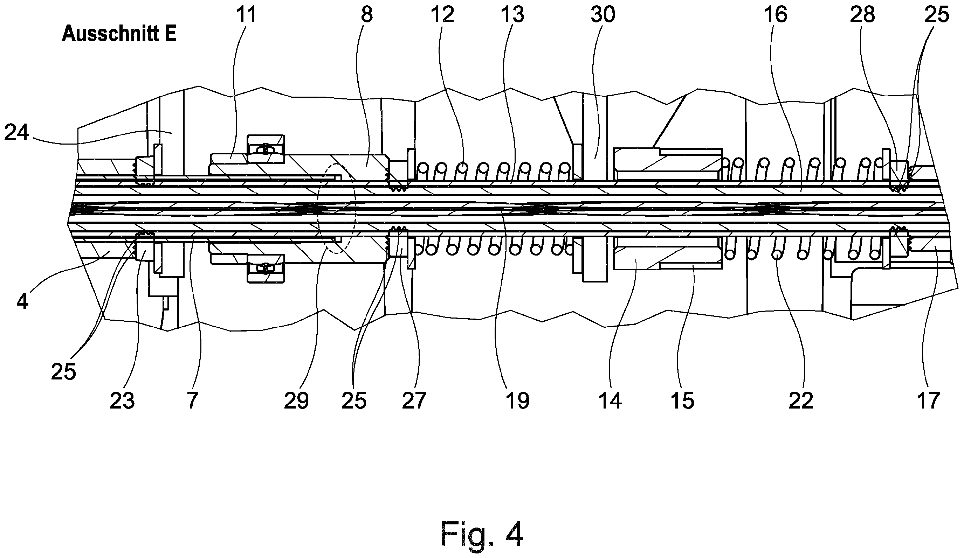

**Fig. 1** zeigt zunächst eine Perspektivansicht eines Instruments für ein robotisches Operationssystem. Das Instrument verfügt über ein Gehäuse 1, welches hier von der Oberseite gezeigt ist. Mit der - hier nicht sichtbaren - Unterseite wird es über eine Sterilschleuse reversibel mit einer Instrumenten-Antriebseinheit verbunden. Bei dem Instrument handelt es sich in der Regel um einen Einweg-Artikel. Aus dem Gehäuse 1 ragt ein Instrumentenschaft, hier mit einem äußeren Schaft 2, hervor. Der äußere Schaft 2 ist an einem distalen Ende mit einem Effektor 3 verbunden und an einem proximalen Ende mit dem Gehäuse 1. Je nachdem, um welche Art von Effektor 3 es sich handelt, müssen verschiedene Bewegungen realisiert werden, wobei die Bewegungen mittels der Instrumenten-Antriebseinheit erzeugt werden. Handelt es sich beispielsweise um ein Greifelement, so müssen Drehungen um die eigene Achse, eine Abwinklung aus der Längsachse des Instrumentenschafts sowie das Öffnen und Schließen des Maulteils realisiert werden. Dies soll im Folgenden anhand eines Längsschnittes A-A durch das Instrument, wie er in **Fig. 2****,** welche das Instrument von oben zeigt, angedeutet ist, näher erläutert werden. **Fig. 3A** zeigt einen Ausschnitt des Längsschnittes mit dem Effektor 3, **Fig. 3B** zeigt einen Ausschnitt des Längsschnittes mit dem Gehäuse 1. **Fig. 4** zeigt einen in **Fig. 3B** mit E markierten Ausschnitt vergrößert.

**Fig. 3A** zeigt das distale Ende des Instruments mit dem Effektor 3, bei dem es sich hier um ein Greifelement, beispielsweise eine Pinzette handelt. Dies ist jedoch nur beispielhaft zu verstehen, ebenso lassen sich als Effektoren 3 auch Klammern oder Scheren oder beliebige andere Effektoren, welche für die laparoskopische Chirurgie anwendbar sind, verwenden. Auch Effektoren mit weniger Freiheitsgraden, wie beispielsweise Haken, kommen infrage. Der äußerer Schaft 2 ist am proximalen Ende über ein - in **Fig. 3B** gezeigtes - erstes Koppelelement 4 mit dem Gehäuse 1 verbunden. Das erste Koppelelement 4 ist dabei mit dem äußeren Schaft 2 fest verbunden, beispielsweise verklebt. Dabei erfolgt die Verbindung des äußeren Schaftes 2 mit dem Gehäuse 1 in der Weise, dass der äußere Schaft 2 um seine Längsachse rotiert werden kann, es handelt sich also um eine rotatorische Verbindung, wozu das erste Koppelelement 4 in einem Lager 5 gelagert ist. Um die Rotation zu realisieren, greift der Antrieb der Instrumenten-Antriebseinheit in ein dazu korrespondierendes erstes rotatorisch angetriebenes Element ein, welches fest mit dem ersten Koppelelement 4 verbunden oder an diesem ausgebildet ist. Im vorliegenden Beispiel ist dem ersten Koppelelement 4 ein erster Zahnkranz 6 aufgeprägt, in den das entsprechende Antriebselement der Antriebseinheit eingreift. Durch Drehung des äußeren Schaftes 2 wird das Instrument um die Längsachse des äußeren Schaftes 2 rotiert.

Das Instrument umfasst außerdem einen ersten inneren Schaft 7, welcher auch als Schwenkrohrschaft bezeichnet wird. Der erste innere Schaft 7 ist teilweise innerhalb des äußeren Schaftes 2 angeordnet und relativ zum äußeren Schaft 2 beweglich - hier translatorisch entlang der Längsachse verschiebbar - gelagert. In anderen Ausführungen insbesondere mit anderen Effektoren kann der erste innere Schaft 7 um die Längsachse rotierbar gelagert sein. Auch der erste innere Schaft 7 ist an einem distalen Ende mit dem Effektor 3 verbunden. An einem proximalen Ende ist der erste innere Schaft 7 fest mit einem zweiten Koppelelement 8 verbunden. Der erste innere Schaft 7 kann in Längsrichtung des äußeren Schaftes 2, welcher auch als Instrumentenschaft bezeichnet wird, translatorisch bewegt werden, um den Instrumentenkopf, d.h. den Effektor 3, zu verschwenken. Das erste Koppelelement 4 dient bezüglich der translatorischen Bewegung des ersten inneren Schaftes 7 auch als Führung. Der Effektor 3 steht dazu über einen Zughebel 9 mit dem distalen Ende des ersten inneren Schaftes 7 in Verbindung. Wird der erste innere Schaft 7 translatorisch in Richtung des proximalen Endes gezogen, so wird der Effektor 3 verschwenkt und ein Biegeelement 10 seitlich gebogen. Zur Übertragung der translatorischen Bewegung von der Antriebseinheit auf den ersten inneren Schaft 7 dient ein erstes Eingriffelement 11 am zweiten Koppelelement 8, mit welchem der erste innere Schaft 7 fest verbunden ist. Die Rückstellung in die Ausgangslage mit axialer Ausrichtung des Effektors 3 wird durch ein erstes axiales Rückstellfederelement 12 erleichtert; das erste axiale Rückstellfederelement 12 übt auf das zweite Koppelelement 8 bei dessen translatorischer Verschiebung aus einer Grundstellung heraus eine dieser Verschiebung entgegengesetzte Federkraft aus. Zwischen dem ersten Koppelelement 4 und dem zweiten Koppelelement 8 ist ein optionales erstes Führungselement 24 angeordnet, in welchem der erste innere Schaft 7 zusätzlich geführt wird.

Teilweise innerhalb des ersten inneren Schaftes 7 ist ein zweiter innerer Schaft 13 angeordnet, der ebenfalls relativ zum äußeren Schaft 2 und zum ersten inneren Schaft 7 beweglich gelagert ist. Im hier gezeigten Beispiel ist der zweite innere Schaft 13 relativ zum äußeren Schaft 2 und zum ersten inneren Schaft 7 um die Achse, d. h. die Längsachse des Instrumentenschafts bzw. des äußeren Schaftes 2, rotierbar gelagert, der zweite innere Schaft 13 wird in diesem Fall auch als Drehrohrschaft bezeichnet. In anderen Ausgestaltungen des Instruments bzw. der Effektoren kann der zweite innere Schaft 13 auch translatorisch beweglich sein. Der zweite innere Schaft 13 ist an einem distalen Ende mit dem Effektor 3 und an einem proximalen Ende fest mit einem dritten Koppelelement 14 verbunden. Die Verbindung ist vorzugsweise stoffschlüssig, kann aber auch formschlüssig und/oder kraftschlüssig erfolgen. Das zweite Koppelelement 8 umschließt dabei den zweiten inneren Schaft 13 in einem zweiten Übergangsbereich 29 am proximalen Ende des ersten inneren Schaftes 7, wobei die Beweglichkeit des zweiten inneren Schaftes 13 gegenüber dem zweiten Koppelelement 8 bzw. dem ersten inneren Schaft 7 nicht beeinträchtigt wird. Das Umschließen erfolgt möglichst passgenau, um ein Eindringen von Flüssigkeit oder Gewebe in das Gehäuse 1 möglich zu verhindern. Gleiches gilt in Bezug auf das Umschließen des ersten inneren Schaftes 7 durch das erste Koppelelement 4. Um den zweiten inneren Schaft 12 rotatorisch zu bewegen, ist am dritten Koppelelement 14 ein weiteres rotatorisch angetriebenes Element, hier ein zweiter Zahnkranz 15 ausgebildet, der durch einen entsprechenden Antrieb in der Instrumenten-Antriebseinheit in Rotation um die Längsachse versetzt werden kann. Der zweite innere Schaft 13 ist mit der Schraubenfeder 10 drehfest verbunden und überträgt die rotatorische Bewegung auf diese. Zwischen dem zweiten Koppelelement 8 und dem dritten Koppelelement 14 ist ein optionales zweites Führungselement 30 angeordnet, in welchem der zweite innere Schaft 13 geführt wird. Ein proximales Ende des ersten axialen Rückstellfederelements 12 liegt dabei am zweiten Führungselement 30 an oder ist daran befestigt, um dem ersten axialen Rückstellfederelement 12 einen entsprechenden Halt zu geben.

Schließlich umfasst das Instrument auch einen dritten inneren Schaft 16, welcher teilweise innerhalb des zweiten inneren Schaftes 13 angeordnet ist und relativ zum äußeren Schaft 2, zum ersten inneren Schaft 7 und zum zweiten inneren Schaft 13 beweglich gelagert ist. Im hier gezeigten Beispiel ist der dritte innere Schaft 16 relativ zu den anderen Schäften 2, 7 und 13 translatorisch entlang der Achse, d. h. der Längsachse des äußeren Schaftes 2 bzw. der Instrumentenlängsachse, verschiebbar gelagert. In Abhängigkeit von einem gewählten Effektor kann der dritte innere Schaft 16 abweichend auch rotatorisch gelagert sein. Auch können gegebenenfalls einer oder mehrere der inneren Schäfte 7, 13 oder 16 wegfallen.

Der dritte innere Schaft 16 ist an einem distalen Ende ebenfalls mit dem Effektor 3 und an einem proximalen Ende fest mit einem vierten Koppelelement 17 verbunden; die Verbindung ist vorzugsweise stoffschlüssig, kann aber auch auf andere Weise erfolgen. Das vierte Koppelelement 17 umschließt den zweiten inneren Schaft 13 in einem dritten Übergangsbereich 31 am proximalen Ende des zweiten inneren Schaftes 13. Die von der Instrumenten-Antriebseinheit erzeugte translatorische Bewegung wird über ein am vierten Koppelelement 17 ausgebildetes zweites Eingriffelement 18 auf dieses übertragen. Das zweite Eingriffelement 18 ist hier als umlaufende Außennut ausgebildet, deren sich in radialer Richtung erstreckende Begrenzungsflächen als Anschlagflächen für den Antrieb dienen. Innerhalb des auch als Greifrohrschaft bezeichneten dritten inneren Schaftes 16 befindet sich ein Betätigungselement für den Effektor 3, hier ein Zugdraht 19, mit dem eine Greif- oder Schneidbewegung des Effektors 3 ausgelöst werden kann. Über die Instrumenten-Antriebseinheit wird der Zugdraht 19 zum Öffnen der Schenkel des Greifelements des Effektors 3 in die distale Richtung verschoben. Mittels eines Hebelelements 20 lässt sich das Greifelement manuell öffnen, beispielsweise bei einem Ausfall des Systems oder bei Vorliegen anderer Fehler, die eine Öffnung über die Instrumenten-Antriebseinheit mittels der Steuerung verhindern. Das Hebelelement wird dazu im Uhrzeigersinn um eine Drehachse, die senkrecht zur Zeichenebene steht, bewegt, und stößt gegen ein Abschlusselement 21, welches stoffschlüssig mit dem vierten Koppelelement 17 verbunden ist und dieses fluiddicht abdichtet. Der Zugdraht 19 ist hier im Abschlusselement 21 befestigt, kann aber auch direkt am vierten Koppelelement 17 befestigt sein. Zum Schließen des Greifelements wird der Zugdraht 19 in die entgegengesetzte, proximale Richtung bewegt. Um einen sicheren und festen Griff zu gewährleisten, wird auf den Zugdraht 19 bzw. das vierte Koppelelement 17 zusätzlich von einem zweiten axialen Rückstellefederelement 22 eine Schubkraft auf das vierte Koppelelement 17 ausgeübt; beim Öffnen wird das zweite axiale Rückstellfederelement 22 gestaucht. Im vorliegenden Beispiel ist das zweite axiale Rückstellefederelement 22 an dem axial fest positionierten dritten Koppelelement 14 befestigt.

Der äußere Schaft 2, der erste innere Schaft 7, der zweite innere Schaft 13 und der dritte innere Schaft 16 sind dabei koaxial angeordnet und untereinander so gekoppelt, dass eine Drehbewegung übertragbar ist. Wird also der äußere Schaft 2 gedreht, so drehen sich der erste innere Schaft 7, der zweite innere Schaft 13 und der dritte innere Schaft 16 gleichermaßen, so dass ihre relativen Stellungen zueinander gleichbleiben. Wird hingegen nur der zweite innere Schaft 13 rotiert, so drehte sich nur der dritte innere Schaft 16 mit.

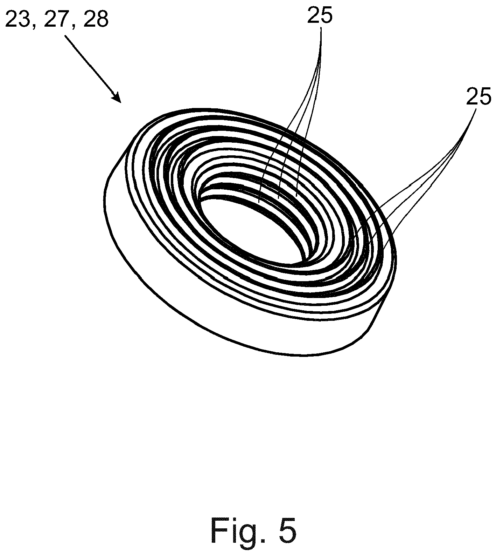

Mit passgenauen Abmessungen der Koppelelemente 4, 8, 14, 17 in den Übergangsbereichen, wo sie jeweils den nächsten innenliegenden Schaft umschließen, lässt sich der Übertritt von Körperflüssigkeiten und Gewebe in das Gehäuse 1 zwar im Wesentlichen unterdrücken, jedoch nicht vollständig ausschließen. Um den Übertritt von Körperflüssigkeiten in das Gehäuse 1 effektiv zu vermeiden, umfasst das Instrument daher mehrere Dichtungselemente. Ein erstes Dichtungselement 23 umschließt den ersten inneren Schaft 7 und dichtet diesen gegen den äußeren Schaft 2 ab. Im vorliegenden Beispiel wird der erste innere Schaft 7 im ersten Führungselement 24 geführt, welches zwischen dem ersten Koppelelement 4 und dem zweiten Koppelelement 8 angeordnet ist. Das erste Dichtungselement 23 ist zwischen dem ersten Koppelelement 4 und dem ersten Führungselement 24 angeordnet. Es wird von diesen beiden Elementen axial fixiert, da das erste Führungselement 24 fest mit dem Gehäuse 1 verbunden ist und auch das erste Koppelelement 4 in axialer Richtung fixiert ist. Diese Positionierung des ersten Dichtungselements 23 ermöglicht eine einfache Nachrüstung bestehender Systeme sowie eine Modifikation des Herstellungsprozesses, ohne dass sämtliche Werkzeuge für die Fertigung, beispielsweise Spritzgusswerkzeuge, modifiziert werden müssten. Das Dichtungselement 23 ist ringförmig ausgebildet. In dem in **Fig. 4** gezeigten Beispiel weist das erste Dichtungselement 23 einen rechteckförmigen Querschnitt auf. **Fig. 5** zeigt das erste Dichtungselement 23 im Detail in einer Perspektivansicht. An den Seiten, die bei Betrieb in Richtung der Koppelelemente und zum jeweiligen Schaft, also in Richtung der abzudichtenden Stellen weisen, sind umlaufend geschlossene, im Querschnitt zahnförmige, elastische Wälle oder Dichtlamellen 25 ausgebildet, die eine Reihe von hintereinander angeordneten Flüssigkeitsbarrieren bilden und so den Effekt der Abdichtung weiter erhöhen.

Alternativ kann auf das erste Führungselement 24 auch verzichtet werden und das erste Dichtungselement 23 kann direkt an einem proximalen Ende des ersten Koppelelements 4 fixiert werden, beispielsweise stoffschlüssig. In einer weiteren alternativen Ausgestaltung kann das erste Dichtungselement 23 auch innerhalb des ersten Koppelelements 4 in einem ersten Übergangsbereich 26 angeordnet sein. Dort wird es axial vom proximalen Ende des äußeren Schaftes 2 und vom ersten Koppelelement 4 fixiert. In keinem der Fälle ist eine wesentliche Modifikation der Werkzeuge und des Herstellungsprozesses für das Instrument notwendig, abgesehen davon, dass in einem zusätzlichen Schritt das erste Dichtungselement 23 integriert werden muss.

Das erste Dichtungselement 23 dichtet den ersten inneren Schaft 7 gegen den äußeren Schaft 2 ab. Zur Abdichtung des zweiten inneren Schaftes 13 gegen den ersten inneren Schaft 7 ist ein zweites Dichtungselement 27 vorgesehen, welches den zweiten inneren Schaft 13 umschließt. Zur Abdichtung des zweiten inneren Schaftes 13 gegen den dritten inneren Schaft 16 ist ein drittes Dichtungselement 28 vorgesehen, welches ebenfalls den zweiten inneren Schaft 13 umschließt, da es am distalen Ende des vierten Koppelelements 17 angeordnet ist. Alle drei Dichtungselemente 23, 27 und 28 können ähnlich aufgebaut sein. Als Material kommt beispielsweise Silikon, insbesondere biokompatibles Silikon, infrage.

Das zweite Dichtungselement 27 ist hier zwischen dem ersten axialen Rückstellfederelement 12 und dem zweiten Koppelelement 8 angeordnet und wird von diesen eingespannt, eine zusätzliche Fixierung am ersten axialen Rückstellfederelement 12 und/oder am zweiten Koppelelement 8 ist nicht nötig, jedoch möglich. Bei einer stoffschlüssigen Verbindung muss darauf geachtet werden, einen Klebstoff zu verwenden, welcher nicht zu einem Aushärten des Materials, aus dem die Dichtungselemente gefertigt sind, führt, da dies die Abdichtungswirkung verringern könnte. Vorzugsweise werden die Dichtungselemente daher, sofern eine stoffschlüssige Fixierung vorgesehen ist, an denjenigen Seiten fixiert, an denen kein Flüssigkeitsdurchtritt zu erwarten ist. Im Falle des zweiten Dichtungselements 27 käme daher insbesondere eine Fixierung am ersten axialen Rückstellfederelement 12 infrage, und im Falle des dritten Dichtungselements 28 eine Fixierung am zweiten axialen Rückstellfederelement 22. Alternativ kann das zweite Dichtungselement 27 auch ausschließlich an einem proximalen Ende des zweiten Koppelelements 8 fixiert sein. In einer weiteren alternativen Ausgestaltung kann das zweite Dichtungselement 27 auch innerhalb des zweiten Koppelelements 8 im zweiten Übergangsbereich 29 angeordnet sein, wo es axial von dem proximalen Ende des ersten inneren Schaftes 7 und dem zweiten Koppelelement 8 fixiert ist.

Das vorangehend beschriebene Instrument ist gegenüber bekannten Instrumenten ähnlicher Bauart aufgrund der Verwendung von Dichtungselementen besser abgedichtet. Die Anordnung der Dichtungselemente kann in bereits bestehende Instrumentenkonfigurationen ohne wesentliche Änderungen übernommen werden. Durch die Verwendung von mehreren Dichtungselementen und deren Verteilung entlang der Längsachse an den Übergangsstellen bzw. in den Übergangsbereichen zwischen den Schäften können sämtliche Stellen, an denen Möglichkeit für einen Flüssigkeitsaustritt in das Gehäuse besteht, effektiv abgedichtet werden. Auf eine zusätzliche Befestigung der Dichtungselemente kann verzichtet werden, da diese in ihren axialen Positionen durch bereits vorhandene Rückstellfederelemente, Koppelelemente und/oder Führungselemente fixiert sind. Sowohl bei rotatorischer als auch bei translatorischer Bewegung können die Schäfte unter den Dichtungselementen gleiten, so dass diese nicht ungewollt verschoben werden.

### Bezugszeichenliste

- 1: Gehäuse
- 2: äußerer Schaft
- 3: Effektor
- 4: erstes Koppelelement
- 5: Lager
- 6: erster Zahnkranz
- 7: erster innerer Schaft
- 8: zweites Koppelelement
- 9: Zughebel
- 10: Biegeelement
- 11: erstes Eingriffelement
- 12: erstes axiales Rückstellfederelement
- 13: zweiter innerer Schaft
- 14: drittes Koppelelement
- 15: zweiter Zahnkranz
- 16: dritter innerer Schaft
- 17: viertes Koppelelement
- 18: zweites Eingriffelement
- 19: Zugdraht
- 20: Hebelelement
- 21: Abschlusselement
- 22: zweites axiales Rückstellfederelement
- 23: erstes Dichtungselement
- 24: erstes Führungselement
- 25: Dichtlamelle
- 26: erster Übergangsbereich
- 27: zweites Dichtungselement
- 28: drittes Dichtungselement
- 29: zweiter Übergangsbereich
- 30: zweites Führungselement
- 31: dritter Übergangsbereich

## Patentansprüche

1. Instrument für ein robotisches Operationssystem umfassend
- ein Gehäuse (1),
- einen äußeren Schaft (2), welcher an einem distalen Ende mit einem Effektor (3) und an einem proximalen Ende mit dem Gehäuse (1) über ein fest mit dem äußeren Schaft (2) verbundenes erstes Koppelelement (4) rotatorisch zur Rotation des äußeren Schaftes (2) um eine Achse verbunden ist,
- einen ersten inneren Schaft (7), welcher teilweise innerhalb des äußeren Schaftes (2) angeordnet und relativ zum äußeren Schaft (2) beweglich gelagert ist, und welcher an einem distalen Ende mit dem Effektor (3) und an einem proximalen Ende fest mit einem zweiten Koppelelement (8) verbunden ist, **dadurch gekennzeichnet, dass**
- das erste Koppelelement (4) den ersten inneren Schaft (7) in einem ersten Übergangsbereich (26) am proximalen Ende des äußeren Schafts (2) umschließt, und
- ein erstes Dichtungselement (23) den ersten inneren Schaft (7) umschließt, dabei entweder in einer ersten Alternative an einem proximalen Ende des ersten Koppelelements (4) fixiert ist oder in einer zweiten Alternative innerhalb des ersten Koppelelements (4) im ersten Übergangsbereich (26) angeordnet ist und axial von dem proximalen Ende des äußeren Schaftes (2) und dem ersten Koppelelement (4) fixiert ist, und somit den ersten inneren Schaft (7) gegen den äußeren Schaft (2) abdichtet.

2. Instrument nach Anspruch 1, **dadurch gekennzeichnet, dass** der erste innere Schaft (7) relativ zum äußeren Schaft (2) translatorisch entlang der Achse verschiebbar gelagert ist.

3. Instrument nach Anspruch 1, erste Alternative, **dadurch gekennzeichnet, dass** zwischen dem ersten Koppelelement (4) und dem zweiten Koppelelement (8) ein erstes Führungselement (24) angeordnet ist, in welchem der erste innere Schaft (7) geführt wird, und das erste Dichtungselement (23) zwischen dem ersten Koppelelement (4) und dem ersten Führungselement (24) angeordnet und von diesen axial fixiert ist.

4. Instrument nach einem der Ansprüche 1 bis 3, außerdem umfassend
- einen zweiten inneren Schaft (13), welcher teilweise innerhalb des ersten inneren Schafts (7) angeordnet und relativ zum äußeren Schaft (2) und zum ersten inneren Schaft (7) beweglich gelagert ist, und welcher an einem distalen Ende mit dem Effektor (3) und an einem proximalen Ende fest mit einem dritten Koppelelement (14) verbunden ist, wobei
- das zweite Koppelelement (8) den zweiten inneren Schaft (13) in einem zweiten Übergangsbereich (29) am proximalen Ende des ersten inneren Schafts (7) umschließt und
- ein zweites Dichtungselement (27) den zweiten inneren Schaft (13) umschließt, dabei in einer ersten Alternative an einem proximalen Ende des zweiten Koppelelements (8) fixiert ist oder in einer zweiten Alternative innerhalb des zweiten Koppelelements (8) im zweiten Übergangsbereich (29) angeordnet ist und dort axial von dem proximalen Ende des ersten inneren Schaftes (7) und dem zweiten Koppelelement (8) fixiert ist, und somit den zweiten inneren Schaft (13) gegen den ersten inneren Schaft (7) abdichtet.

5. Instrument nach Anspruch 4, **dadurch gekennzeichnet, dass** der zweite innere Schaft (13) relativ zum äußeren Schaft (2) und zum ersten inneren Schaft (7) um die Achse rotierbar gelagert ist.

6. Instrument nach Anspruch 4, erste Alternative, bei dem der erste innere Schaft (7) relativ zum äußeren Schaft (2) translatorisch entlang der Achse verschiebbar gelagert ist, umfassend ein erstes axiales Rückstellfederelement (12) mit einem proximalen und einem distalen Ende, welches auf das zweite Koppelelement (8) bei dessen translatorischer Verschiebung aus einer Grundstellung heraus eine dieser Verschiebung entgegengesetzte Federkraft ausübt, wobei das zweite Dichtungselement (27) zwischen dem zweiten Koppelelement (8) und dem ersten axialen Rückstellfederelement (12) angeordnet und von diesen eingespannt ist.

7. Instrument nach Anspruch 6, umfassend außerdem ein zweites Führungselement (30), welches zwischen dem zweiten Koppelelement (8) und dem dritten Koppelelement (14) angeordnet ist und in welchem der zweite innere Schaft (13) geführt wird, wobei das proximale Ende des ersten axialen Rückstellfederelements (12) am zweiten Führungselement (30) anliegt oder befestigt ist.

8. Instrument nach einem der Ansprüche 4 bis 7, außerdem umfassend
- einen dritten inneren Schaft (16), welcher teilweise innerhalb des zweiten inneren Schafts (13) angeordnet und relativ zum äußeren Schaft (2), zum ersten inneren Schaft (7) und zum zweiten inneren Schaft (13) beweglich gelagert ist, und welcher an einem distalen Ende mit dem Effektor (3) und an einem proximalen Ende fest mit einem vierten Koppelelement (17) verbunden ist, wobei
- das vierte Koppelelement (17) den zweiten inneren Schaft (13) in einem dritten Übergangsbereich (31) am proximalen Ende des zweiten inneren Schafts (13) umschließt und
- ein drittes Dichtungselement (28) an einem distalen Ende des vierten Koppelelements (17) fixiert ist, den zweiten inneren Schaft (13) umschließt und gegen den dritten inneren Schaft (16) abdichtet.

9. Instrument nach Anspruch 8, **dadurch gekennzeichnet, dass** der dritte innere Schaft (16) relativ zum äußeren Schaft (2), zum ersten inneren Schaft (7) und zum zweiten inneren Schaft (13) translatorisch entlang der Achse verschiebbar gelagert ist.

10. Instrument nach Anspruch 8 oder 9, umfassend ein zweites axiales Rückstellfederelement (22), welches mit einem distalen Ende am dritten Koppelelement (14) anliegt oder an diesem befestigt ist und auf das vierte Koppelelement (17) bei dessen translatorischer Verschiebung aus einer Grundstellung heraus eine dieser Verschiebung entgegengesetzte Federkraft ausübt, wobei das dritte Dichtungselement (28) zwischen dem distalen Ende des vierten Koppelelements (17) und dem proximalen Ende des zweiten axialen Rückstellfederelements (22) angeordnet und von diesen eingespannt ist.

11. Instrument nach einem der Ansprüche 8 bis 10, **dadurch gekennzeichnet, dass** in dem dritten inneren Schaft (16) mindestens ein Betätigungselement für den Effektor (3) angeordnet ist, welches als Zugseil oder Zugdraht (19) ausgebildet ist.

12. Instrument nach einem der vorgenannten Ansprüche 1 bis 11, **dadurch gekennzeichnet, dass** das erste Dichtungselement (23) und, falls vorhanden, das zweite Dichtungselement (27) und das dritte Dichtungselement (28) als Dichtringe ausgebildet sind.

13. Instrument nach Anspruch 12, **dadurch gekennzeichnet, dass** die Dichtringe einen rechteckigen Querschnitt aufweisen und an einem inneren Umfang und/oder an mindestens einer Stirnfläche umlaufend geschlossene, im Querschnitt zahnförmige Lamellen ausgebildet sind.

14. Instrument nach einem der vorgenannten Ansprüche 1 bis 13, **dadurch gekennzeichnet, dass** das erste Dichtungselement (23) und, falls vorhanden, das zweite Dichtungselement (27) und das dritte Dichtungselement (28) aus Silikon sind oder Silikon enthalten.
